# EUROPEAN PATENT APPLICATION

(11) **EP 4 113 522 A1**
(43) Date of publication of application: **04.01.2023**
(21) Application number: 21183232.4
(22) Date of filing: 01.07.2021
(51) Int. Cl.: G16B 40/20

(54) **SYSTEM AND METHOD FOR THE IDENTIFICATION OF BIOLOGICAL COMPOUNDS FROM THE GENETIC INFORMATION IN EXISTING BIOLOGICAL RESOURCES**

(71) Applicant: Senckenberg Gesellschaft Für Naturforschung, 60325 Frankfurt Am Main (DE)
(72) Inventor: de Gaudenzi, Luigi, 60386 Frankfurt (DE)
(74) Representative: Stellbrink & Partner Patentanwälte mbB

(57) **Abstract**

The present invention is directed to an identification of biological feature in a biological resource. An analyzing component (10,15) can be configured for testing and/or analyzing the datasets retrieved for the same and/or a compatible and/or a similar biological activity of the features known. A similarity of the datasets to the biologically active features can be preset or pre-defined by a minimum threshold value of similarity. This can be a fixed or a dynamic threshold value. The similarity of the datasets to the biologically active features can be determined by a sliding minimum threshold value of similarity. The sliding minimum threshold value of similarity can set by a machine learning algorithm. This also comprises a change of the threshold value over time as a result of a machine learning training algorithm trained by results of testing and/or analyzing the datasets identified for the biological activity of the features known.

## Description

### Field

The present invention relates to a system and a method for the identification of biological compounds from existing biological resources, using the genetic information from the pharmaceutically and/or chemically active and/or industrially applicable genetic sequences or proteins contained within said biological resources. The biological resources can comprise a collection with a very considerable volume of biological material.

### Introduction

In general, biological resources, such as genetic resources from naturally occurring products and compounds, can have a pharmaceutically and/or chemically and/or industrially active impact onto the onset or the treatment of diseases and can form part or even the substantial component of respective pharmaceuticals. For that reason, biologically interesting substances are attempted to be spotted in nature, e.g. as new antibiotics. This has been done in the past, to an extent that was even considered improper and over-exploiting the genetic resources of certain countries.

For that reason, the Nagoya Protocol on Access to Genetic Resources and the Fair and Equitable Sharing of Benefits Arising from their Utilization (ABS) to the Convention on Biological Diversity, entered in force in 2014. It is a supplementary agreement to the 1992 Convention on Biological Diversity (CBD). The CBD sets the framework for the objective of conservation of biological diversity, stating the sovereign right of the countries of origin to exploit their own resources pursuant to their own environmental policies. The aim of ABS is the implementation of one of the three objectives of the CBD: the fair and equitable sharing of benefits arising out of the utilization of genetic resources, thereby contributing to the conservation and sustainable exploitation of biodiversity. It sets out obligations for its contracting parties to take measures in relation to access to genetic resources, benefit-sharing and compliance.

This shows the great interests and prospects into the mining of genetic resources.

US 2018 0330 281 A1 discloses a platform for a prediction based on extraction of features and observations collected from a large number of (disparate) data sources using machine learning to reinforce quality of collection, prediction and action based on those predictions.

It is directed to an automated data collection from a plurality of sources, an automated processing of data, an automated non-human agent, i.e., AI, for automatically taking actions based on a prediction, an automated reward or punishment identification, i.e., data scoring, an automated machine learning-based improvement of selection features, and an automated feedback of data scoring.

WO 2010 080130 A1 discloses a method for analyzing genetic data, the method comprising input units providing information about association between genetic variants and phenotypes, input units assigned to one of a plurality of clusters based on an assessment of shared genetic variants, and identifying phenotypes that share underlying biological mechanisms. It further discloses receiving input data comprising a plurality of genetic variants, selecting a region or regions of the input data, and for each selected region(s) assigning an input unit. It is directed to the calculating of similarity of input units to a plurality of clusters based on the assessment of share genetic variants. A receiving of input data comprises a plurality of genetic variants, wherein clusters assignments are explored by means of a Markov chain Monte Carlo algorithm.

EP 347 1027 A1 discloses a method for the computer-implemented determination of a data-driven prediction model, the method comprising input and output variables being semantically annotated based on a digital semantic representation having a hierarchical tree structure where each tree in the structure represents an input variable of the data, leaf nodes of the respective tree being the discrete values of the input variable. The method disclosed comprises a recording of discrete values by cutting off hierarchical levels and data modifications are determined for the input data. Those data modifications comprise a training of data by means of a machine learning method, a prediction training by machine learning, determining of a prediction model with high prediction quality by using a semantic model in combination with variable recoding and machine learning, and applying the method to any kind of data, e.g., to medical data or data of a technical system.

The before fails to teach using a computer-implemented method or system for analysis, prediction and/or valuation of biological or genetic resources of practical impact.

### Summary

In light of the above, it is an object of the present invention to overcome or at least alleviate the shortcomings of the prior art.

The present invention is directed to a system and a method. Wherever one of those categories and its features are discussed the same applies for the other category and its features mutatis mutandis.

The invention relates to a method and a system for an identification of biological feature in a biological resource. It can comprise a resource database that is configured for providing datasets. The database and the datasets are particularly configured for comprising biological data. It can comprise a genetic data base with genetic data of a plurality and even a mass of individual samples. The genetic data can comprise genomic data, genomic sub-data, data regarding relevant mutations, sequences, CNV (*copy number variation*) or SNP (*single nucleotide polymorphism*) data etc.

The present invention can comprise an analyzing component. In can be provided integral or can comprise several parts, e.g., a computing device, a comparator, an analyzer component etc. It can be configured for analyzing similarity, such as bio similarity, and/or compatibility of the datasets with biologically active features known. This can refer to the applicability of those features for pharmaceutical, chemical and/or other industrial use. This analysis is configured for identifying the datasets that have the same, a compatible and/or have a minimum similarity to the biologically active features.

The resource database can be configured for providing a plurality of datasets. A computing component can be configured for providing at least one first profile relevant for retrieving compatible datasets to at least one preset feature. This can be realized in order to be able to quickly and efficiently crawl through the database for reducing the time and computer power for reducing the number of potential samples. As the functional similarity or combability can be a complex task to analyze, a necessary common attribute, such as SNPs is known to be necessary for the functionality or purpose looked for, can be taken as a basis or first profile for a first limiting search. The computing component can be also configured for automatically approaching the resource database and retrieving datasets from it according to the first profile. This means that one or more databases can be approached and their biological data can be checked or analyzed for the first profile. The computing device or a component thereof can be further configured for training the first profile on the basis of the datasets retrieved.

A system or a method for an identification of a biological feature in a biological resource can also comprise a resource database configured for providing a plurality of datasets and a computing component configured for providing at least one first profile relevant for retrieving compatible datasets to at least one preset feature, wherein the first profile has been trained over time, and for automatically approaching the resource database and retrieving datasets from it according to the first profile. This is focusing on the training of a profile over time and in order to improve the success or efficiency of the method and system. Everything mentioned before with respect to a first aspect of the invention applies mutatis mutandis to this and the next aspects.

The present invention also comprises any combination of any of the aspects, systems and/or methods.

An analyzing component can be configured for testing and/or analyzing the datasets retrieved for the same and/or a compatible and/or a similar biological activity of the features known. The testing and/or analyzing can be performed in one or more steps or components and can also comprise different kinds of tests.

The similarity of the datasets to the biologically active features can be preset or pre-defined by a minimum threshold value of similarity. This can be a fixed or a dynamic threshold value. The similarity of the datasets to the biologically active features can be determined by a sliding minimum threshold value of similarity. The sliding minimum threshold value of similarity can set by a machine learning algorithm. This also comprises a change of the threshold value over time as a result of a machine learning training algorithm trained by results of testing and/or analyzing the datasets identified for the biological activity of the features known.

The computing component can be configured for automatically approaching the resource database and retrieving datasets from it. The retrieving of the datasets can be based on a first profile and selecting a plurality of first datasets being at least similar to or comprising this first profile. The comparator can be configured for selecting one or more preferred datasets among the first datasets and automatically analyzing the preferred datasets according to at least a second profile. The second profile can be more complex which may result in more computing power but more precise or fitting results.

The first profile can comprise a subset of a sequence of the active feature known. This can be necessary SNPs or genetic subsequences.

The first profile can be less complex than the second profile. Pharmaceutically and/or chemically active and/or industrially applicable features can be provided by an application database. This can be a separate database or even a network or distributed network of different databases. The application database can comprise data of pharmaceutically and/or chemically active and/or industrially applicable features known.

These features known can be any structure, sequence etc. that are known for a pharmaceutical and/or chemical and/or industrial applicability.

The computing component can be configured for computing of the datasets from the resource database by automatically gathering dataset by dataset and/or sets of datasets from the resource database.

The datasets from the resource database can be sequentially analyzed with the pharmaceutically and/or chemically active and/or industrially applicable features by an analyzing component.

The datasets from the resource database can be analyzed with the pharmaceutically and/or chemically active and/or industrially applicable features in parallel, at least in part, by an analyzing component. The analyzing can be prioritized according to pre-set features.

The analyzing and providing for further use the similarity can have at least a preset threshold value.

The threshold can be a dynamic threshold that can vary according to a training of the analyzing step.

The datasets provided can be used for the formulation of pharmaceutically and/or chemically active and/or industrially applicable compounds. Formulation can comprise the provision of a product or sub-product with the biological data or feature retrieved. This can be done by machine learning or supervised machine learning.

There can also be provided an automatically analyzing of the datasets on the bases of unsupervised machine learning. This can be realized by the computing component. It can also perform an automatic analyzing the datasets on the bases of at least two approaches.

The system and the method can also realize a step of repeatedly selecting datasets and weighing the profiles of the first and second approaches and classifiers from the supervised machine learning on the basis of the profiles and classifiers and providing an actual retrieving profile combining the weighted profiles and classifiers.

A classifier can comprise data or information that represent the quality and/or quantity of the probable or actual applicability of datasets for the application searched for.

Any database mentioned can be a local database, a remote database, a cloud database and any combination thereof. The system can also realize an automatic analyzing of the preferred datasets according to the profiles of the first and second approaches and the classifiers of the supervised machine learning.

The system can be configured for repeatedly selecting datasets weighing the profiles of the first and second approaches and the classifiers on the basis of the profiles and classifiers and providing an actual retrieving profile combining the weighted profiles and classifiers.

The first approach can comprise a first model and the second approach a second model and the second model can be different to the first model. The first approach profile can comprise a numerical representation of the analysis of the originating data and/or the selected dataset(s) according to the first model.

The second approach profile can comprise a numerical representation of the analysis of the originating data and/or the selected dataset(s) according to the second model. The first approach can comprise a content analytics model and the first approach profile can comprise a first approach multidimensional representation.

The second approach can comprise a past distribution statistics model and the second approach profile comprises a second approach multidimensional representation.

The first and second approach multidimensional representations can be vectorized. A further step of retrieving datasets from the database on the basis on either one, two or all of the three models can be provided as well.

A weighing and/or gradually combining the three models on the basis of the last result of the selection component can also be provided. The analyzing component can be activated by a soft key for refreshing the analysis.

An activating of the analyzing by a soft key for refreshing the analysis after the selecting component can be provided.

The datasets each can comprise at least one of datasets, callable units, metadata and content data and any combination thereof.

The inputting of data can be performed automatically or semi-automatically. The inputting of data can be supported by a graphical user interface (GUI).

The inputting of data can be performed by initiating the pulling of data from a data base by entering a code.

The database can comprise more than a million datasets, preferably more than 10 million datasets and most preferably more than 100 million datasets.

The machine learning establishes classifiers on the basis of the profiles by forming random decision trees on the basis of the features of the profiles and determining relevant decision trees from less relevant decision trees on the basis of profiles relevant for retrieving compatible datasets to originating data.

Any retrieving of compatible datasets can trigger the analyzing component to analyze the originating data or any subsequent originating data on the basis of the first approach, the second approach and the classifiers.

The datasets each can comprise at least one of datasets, callable units, metadata and content data and any combination thereof. The inputting of originating data or grading datasets automatically or semi-automatically can also be provided.

An inputting component can initiate the system upon entry of an individual password. A repeating of the initiating of the retrieving of compatible datasets any time on the most recent corresponding profile(s) in the analytics database can be provided and/or a controlling of a plurality of inputting components so that they are configured or can be configured upon entry of a code to share data in the analytics database assigned to the inputting components.

The system and method can also provide a repeating of the retrieving of compatible datasets based on the profiles in the analytics database and/or a repeating of the retrieving compatible data by another inputting component than the previous one.

The originating data can comprise metadata and content data. The originating data can comprise a code that is part of the metadata and wherein the database can comprise the other metadata and/or content data that corresponds to the code of the originating data.

Pulling the other metadata and/or the content data from the database on the basis of the code can be provided as well.

The datasets can each comprise metadata and content data. The features of the profiles can correspond to portions of the metadata of the datasets.

The originating data can be provided by the inputting component in encoded fashion in a code and the analyzing component can be adapted to gather the complete originating data from the database based on that code.

The code can comprise an ID number, such as a document number, and the complete originating data can comprise a document. The complete originating data can comprise meta data and/or content data.

The metadata can comprise more than 5 fields, preferably more than 10 fields, even more preferably more than 50 fields. The metadata can comprise less than 100 fields, preferably less than 90 fields, even more preferably less than 70 fields.

The analyzing can comprise a multidimensional analysis (MDA).

The first profile and/or the second profile can be (a) vectorized profile(s) and the search in the general architecture is based on that vectorized profile(s).

The plurality of the datasets is sorted according to their relevance and/or weight and/or neighborhood and/or their similarity to the originating data or most recent originating data.

A displaying of a representative summary of the plurality of the datasets sorted according to their relevance and/or neighborhood and/or their similarity to the originating data or most recent originating data can be provided.

A de-selecting and/or weighing of the datasets upon one or more clicks by the inputting component in pre-defined fields can be realized as well. A displaying of a representative summary of the plurality of the datasets sorted according to their relevance and/or neighborhood and/or their similarity to the originating data or most recent originating data and providing the option to out select datasets and/or to provide a quantitative value to their relevance can be provided. This quantitative value can amount from 0 to 3 or from 0 to 4. A further value representing a de-selecting of a dataset can be added as well.

A representative summary of the plurality of datasets can comprise a plurality of metadata of the respective datasets. The inputting component can be further configured to allow an expansion of any parts of the datasets upon a click by the inputting component. Also, a billing for the retrieving of datasets can be provided.

The present invention can also comprise an evaluating component for retrieving of datasets corresponding to their quality and/or quantity and/or compatibility and/or similarity to a biological feature.

A sequencing component for sequencing genetic samples and/or a sequencing analyzer for analyzing sequences of genetic samples can also be provided. The present invention can further comprise a high throughput sequencing component for sequencing genetic samples with high throughput.

Further, a metagenomics component can be provided as well for providing and or sequencing metagenomics samples, such as environmental samples taken from soil, water and/or air.

The following terms and meanings are used throughout the following description:
Red biotechnology: Devoted to medicine and human health and responsible, according to the Biotechnology Innovation Organization (BIO), for the development of more than 250 vaccines and medications such as antibiotics, regenerative therapies and the production of artificial organs;
Green biotechnology: Devoted to the development of agriculture, understood, for example, to provide the technology to fight pests and nourish crops and strengthen them against microorganisms and extreme weather events, such as droughts and frosts;
White biotechnology: meaning industrial biotechnology, this field works, for example, to improve manufacturing processes, the development of biofuels and other technologies to make industry more efficient and sustainable;
Yellow biotechnology: Nutritional biotechnology, focused on food production and, for example, it carries out research to reduce the levels of saturated fats in cooking oils or to make crops resistant to insect attacks;
Blue biotechnology: This exploits aquatic resources, marine and freshwater, for example to obtain aquaculture, cosmetics and health care products, in addition, it is the branch most widely used to obtain biofuels from certain microalgae;
Grey biotechnology: concerning natural capital, its purpose is the conservation and restoration of contaminated natural ecosystems through bioremediation processes; and
Gold biotechnology: also known as bioinformatics it is responsible for obtaining, storing, analyzing and separating biological information, especially that related to DNA and amino acid sequences. The present invention can be understood as relating to the field of gold biotechnology.

The term "component" is intended to comprise a unitary entity and/or one or more entities that are distributed in location. Thus, a component can comprise an integral element as well as an assembly of same and/or different elements.

The term "biological feature" comprises the applicability of a feature from or in any of the before listed fields of biotechnology.

Machine learning (ML) comprises computer algorithms that improve automatically through experience and by the use of data. It is seen as a part of artificial intelligence. Machine learning algorithms or approaches build a model based on sample data, known as "training data", in order to make predictions or decisions without being explicitly programmed to do so. The term "training" is intended to comprise this kind of machine learning approach.

Supervised learning (SL) is the machine learning task of learning a function that maps an input to an output based on example input-output pairs. It infers a function from labeled training data consisting of a set of training examples. In supervised learning, each example is a pair consisting of an input object (typically a vector) and a desired output value (also called the supervisory signal). A supervised learning algorithm analyzes the training data and produces an inferred function, which can be used for mapping new examples.

Unsupervised learning (UL) is a type of algorithm that learns patterns from untagged data. The machine is forced to build a compact internal representation of its world and then generate imaginative content. In contrast to supervised learning (SL) where data is tagged by a human, e.g., as "car" or "fish" etc., UL exhibits self-organization that captures patterns as neuronal predilections or probability densities. The other levels in the supervision spectrum are reinforcement learning where the machine is given only a numerical performance score as its guidance, and semi-supervised learning where a smaller portion of the data is tagged. Two broad methods in UL are Neural Networks and Probabilistic Methods.

Deep learning (also known as deep structured learning) is part of a broader family of machine learning methods based on artificial neural networks with representation learning. Learning can be supervised, semi-supervised or unsupervised.

Deep-learning architectures such as deep neural networks, deep belief networks, graph neural networks, recurrent neural networks and convolutional neural networks have been applied to fields including computer vision, speech recognition, natural language processing, machine translation, bioinformatics, drug design, medical image analysis, material inspection and board game programs, where they have produced results comparable to and in some cases surpassing human expert performance.

The adjective "deep" in deep learning refers to the use of multiple layers in the network.

Explainable AI (XAI) or explainable artificial intelligence can be applied and is understood as artificial intelligence (AI) in which the results of the solution can be understood by humans.

The before definitions have been taken from Wikipedia on June 22, 2021. These are herein incorporated by reference including further information contained therein.

The term "classifier" is intended to comprise a value corresponding to the quality and/or quantity for fulfilling a particular function or biological feature looked for.

It is an aspect of the present invention to select known biologically active structures or features from biotechnological processes, methods and genetic information. The latter comprises genetic sequences that are used for economically relevant products and treatments. The term products and treatments comprise the before colored biotech approaches, such as red, green, white, yellow, blue, grey and gold biotechnologies. As a biological recourse data is taken for existing and/or new biological data that is already comprised in biological data collections, in gene banks like the "Svalbard Global Seed Vault", internal databases of biotechnology companies or research institutes, natural history museums like the one of the Senckenberg Institute, and/or new data newly collected, such as under the Nagoya Protocol. In the case of sequences or SNPs these can be taken as a basis for the methods and systems etc. according to the present invention.

This selection and analysis and tests etc. can be further taken in order to train and improve the selection and/or prediction of datasets and/or biological data from the resource data base.

The present invention can thus help to better and more systematically and/or efficiently make use of known or newly discovered genetic data and to provide negotiations under the Nagoya Protocol with market relevant data. Companies in the biotechnology sector can obtain information for the planning for their technical developments and incorporate the evaluation of resources into their balance sheets. Regulatory authorities are provided with a control instrument for imposing incentive-based environmental regulations. Research institutions can focus on scientific fields that promise successful tech transfer results.

The invention is further described with the following numbered embodiments.

Below, system embodiments will be discussed. These embodiments are abbreviated by the letter "S" followed by a number. Whenever reference is herein made to "system embodiments", these embodiments are meant.
S1. System for an identification of biological feature in a biological resource comprising:
   a. a resource database (1) configured for providing datasets;
   b. an analyzing component (10,15)
      i. configured for analyzing similarity and/or compatibility of the datasets with biologically active features known; and
      ii. for identifying the datasets that have the same, a compatible and/or have a minimum similarity to the biologically active features.
S2. System for an identification of a biological feature in a biological resource comprising:
   a. a resource database (1) configured for providing a plurality of datasets;
   b. a computing component (10)
      i. configured for providing at least one first profile relevant for retrieving compatible datasets to at least one preset feature; and
      ii. for automatically approaching the resource database (1) and retrieving datasets from it according to the first profile; and
      iii. training the first profile on the basis of the datasets retrieved.
S3. System for an identification of a biological feature in a biological resource comprising:
   a. a resource database (1) configured for providing a plurality of datasets;
   b. a computing component (10)
      i. configured for providing at least one first profile relevant for retrieving compatible datasets to at least one preset feature;
      ii. wherein the first profile has been trained over time;
      iii. and for automatically approaching the resource database (1) and retrieving datasets from it according to the first profile.
S4. System comprising any combination of the preceding system embodiments.
S5. System according to the preceding system embodiment with an analyzing component (10,15) configured for testing and/or analyzing the datasets retrieved for the same and/or a compatible and/or a similar biological activity of the features known.
S6. System according to the preceding system embodiment with an analyzing component (10,15) configured for testing and/or analyzing the datasets retrieved for a similar biological activity of the features known.
S7. System according to any of the preceding system embodiments wherein the similarity of the datasets to the biologically active features is preset by a minimum threshold value of similarity.
S8. System according to any of the preceding system embodiments wherein the similarity of the datasets to the biologically active features is determined by a sliding minimum threshold value of similarity.
S9. System according to any of the preceding system embodiments wherein the similarity of the datasets to the biologically active features is determined by a sliding minimum threshold value of similarity that is set by a machine learning algorithm.
S10. System according to any of the preceding system embodiments wherein the similarity of the datasets to the biologically active features is determined by a sliding minimum threshold value of similarity that is set by a machine learning algorithm trained by results of testing and/or analyzing the datasets identified for the biological activity of the features known.
S11. System according to any of the preceding system embodiments for an identification of a pharmaceutically and/or chemically active and/or industrially applicable biological feature in a biological resource, with the computing component (10) configured for automatically approaching the resource database (1) and retrieving datasets from it.
S12. System according to any of the preceding system embodiments with an analyzing component (10,15) configured for automatically approaching the resource database (1) and retrieving datasets from it according to a first profile and selecting a plurality of first datasets being at least similar to or comprising this first profile.
S13. System according to any of the preceding system embodiments with a comparator (10,15) configured for selecting one or more preferred datasets among the first datasets and automatically analyzing the preferred datasets according to at least a second profile.
S14. System according to any of the preceding system embodiments with a computing component (10) configured for automatically approaching the resource database (1) and retrieving datasets from it according to a first profile and selecting a plurality of first datasets being at least similar to or comprising this first profile and selecting one or more of the first datasets and analyzing the preferred datasets according to at least a second profile.
S15. System according to any of the preceding system embodiments with a computing component (10) configured for automatically approaching the resource database (1) and retrieving datasets from it according to a first profile and selecting a plurality of first datasets being at least similar to or comprising this first profile wherein the first profile comprises a subset of a sequence of the active feature known.
S16. System according to any of the preceding system embodiments with a computing component (10) configured for automatically approaching the resource database (1) and retrieving datasets from it according to a first profile and selecting a plurality of first datasets being at least similar to or comprising this first profile and selecting one or more of the first datasets and analyzing the preferred datasets according to at least a second profile wherein the first profile is less complex than the second profile.
S17. System according to the preceding system embodiment wherein pharmaceutically and/or chemically active and/or industrially applicable features are provided by an application database (2).
S18. System according to the preceding system embodiment wherein the application database (2) comprises data of pharmaceutically and/or chemically active and/or industrially applicable features known.
S19. System according to the preceding system embodiment wherein the application database (2) comprises data of pharmaceutically and/or chemically active and/or industrially applicable features and their applications known.
S20. System according to any of the preceding system embodiments wherein the computing component (10) is configured for computing of the datasets from the resource database (1) by automatically gathering dataset by dataset and/or sets of datasets from the resource database (1).
S21. System according to any of the preceding system embodiments wherein the datasets from the resource database (1) are sequentially analyzed with the pharmaceutically and/or chemically active and/or industrially applicable features by an analyzing component (10,15).
S22. System according to any of the preceding system embodiments wherein the datasets from the resource database (1) are analyzed with the pharmaceutically and/or chemically active and/or industrially applicable features in parallel, at least in part, by an analyzing component (10,15).
S23. System according to any of the preceding system embodiments wherein the analyzing is prioritized according to pre-set features.
S24. System according to any of the preceding system embodiments wherein between the analyzing and providing for further use the similarity has at least a preset threshold value.
S25. System according to any of the preceding system embodiments wherein threshold is a dynamic threshold that can vary according to a training of the analyzing step.
S26. System according to any of the preceding system embodiments wherein the datasets provided are used for the formulation of pharmaceutically and/or chemically active and/or industrially applicable compounds.
S27. System according to any of the preceding system embodiments which is configured for further automatically analyzing the datasets on the bases of supervised machine learning.
S28. System according to any of the preceding system embodiments which is configured for further automatically analyzing the datasets on the bases of unsupervised machine learning.
S29. System according to any of the preceding system embodiments wherein any training and/or machine learning is based on the explainable AI approach (XAI).
S30. System according to any of the preceding system embodiments which is configured for further automatically analyzing the datasets on the bases of at least two approaches.
S31. System according to any of the preceding system embodiment which is configured for further repeatedly selecting datasets and weighing the profiles of the first and second approaches and classifiers from the supervised machine learning on the basis of the profiles and the classifiers and providing an actual retrieving profile combining the weighted profiles and the classifiers.
S32. System according to the preceding system embodiment which is configured for further automatically analyzing the preferred datasets according to the profiles of the first and second approaches and the classifiers of the supervised machine learning.
S33. System according to any of the preceding system embodiments which is configured for further repeatedly selecting datasets weighing the profiles of the first and second approaches and the classifiers on the basis of the profiles and the classifiers and providing an actual retrieving profile combining the weighted profiles and the classifiers.
S34. System according to any of the preceding system embodiments wherein in the first approach is a first model and the second approach is a second model and the second model is different to the first model.
S35. System according to any of the preceding system embodiments wherein the first approach profile comprises a numerical representation of the analysis of the originating data and/or the selected dataset(s) according to the first model.
S36. System according to the preceding system embodiment wherein the second approach profile comprises a numerical representation of the analysis of the originating data (2) and/or the selected dataset(s) (10-12) according to the second model.
S37. System according to any of the preceding system embodiments wherein the first approach comprises a content analytics model and the first approach profile comprises a first approach multidimensional representation.
S38. System according to any of the preceding system embodiments wherein the second approach comprises a past distribution statistics model and the second approach profile comprises a second approach multidimensional representation.
S39. System according to the two preceding system embodiments wherein the first and second approach multidimensional representations are vectorized.
S40. System according to any of the preceding system embodiments which is configured for further retrieving datasets from the database (4) on the basis on either one, two or all of the three models.
S41. System according to the preceding system embodiment which is configured for further weighing or gradually combining the three models on the basis of the last result of the selection component (5).
S42. System according to any of the preceding system embodiments wherein the analyzing component can be activated by a soft key for refreshing the analysis.
S43. System according to any of the preceding system with the further step of activating the analyzing by a soft key for refreshing the analysis after the selecting component (5) has been activated.
S44. System according to the preceding system embodiment wherein the datasets each comprise at least one of datasets, callable units, metadata and content data and any combination thereof.
S45. System according to any of the preceding system embodiments wherein the inputting of data is performed automatically or semi-automatically.
S46. System according to any of the preceding system embodiments wherein the inputting of data is supported by a graphical user interface (GUI).
S47. System according to any of the preceding system embodiments wherein the inputting of data is performed by initiating the pulling of data from a data base by entering a code.
S48. System according to any of the preceding system embodiments wherein the database comprises more than a million datasets, preferably more than 10 million datasets and most preferably more than 100 million datasets.
S49. System according to any of the preceding system embodiments wherein the machine learning establishes classifiers on the basis of the profiles by forming random decision trees on the basis of the features of the profiles and determining relevant decision trees from less relevant decision trees on the basis of profiles relevant for retrieving compatible datasets to originating data.
S50. System according to any of the preceding system embodiments wherein any retrieving of compatible datasets makes the analyzing component (3) to analyze the originating data or any subsequent originating data on the basis of the first approach, the second approach and the classifiers.
S51. System according to any of the preceding system embodiments wherein the datasets each comprise at least one of datasets, callable units, metadata and content data and any combination thereof.
S52. System according to any of the preceding system embodiments steps of inputting originating data or grading datasets automatically or semi-automatically.
S53. System according to the preceding system embodiment wherein an inputting component can initiate the system upon entry of an individual password.
S54. System according to any of the preceding system embodiments with the step of repeating the initiating of the retrieving of compatible datasets any time on the most recent corresponding profile(s) in the analytics database.
S55. System according to any of the preceding system embodiments further comprising controlling a plurality of inputting components so that they are configured or can be configured upon entry of a code to share data in the analytics database (6) assigned to the inputting components.
S56. System according to any of the preceding system embodiments which is configured for further repeating retrieving compatible datasets based on the profiles in the analytics database.
S57. System according to the two preceding system embodiments which is configured for further repeating retrieving compatible data by another inputting component than the previous one.
S58. System according to any of the preceding system embodiments wherein the originating data comprises metadata and content data.
S59. System according to the preceding system embodiment wherein the originating data comprises a code that is part of the metadata and wherein the database comprises the other metadata and/or content data that corresponds to the code of the originating data.
S60. System according to the preceding system embodiment with the step of pulling the other metadata and/or the content data from the database on the basis of the code.
S61. System according to any of the preceding system embodiments wherein the datasets each comprise metadata and content data.
S62. System according to the preceding system embodiment wherein the features of the profiles correspond to portions of the metadata of the datasets.
S63. System according to any of the preceding system embodiments wherein the originating data is provided by the inputting component in encoded fashion in a code and the analyzing component is adapted to gather the complete originating data from the database based on that code.
S64. System according to any of the preceding system embodiments wherein the code is an ID number, such as a document number, and the complete originating data comprises a document.
S65. System according to the preceding system embodiment wherein the complete originating data comprises meta data and content data.
S66. System according to any of the preceding system embodiments wherein the metadata comprises more than 5 fields, preferably more than 10 fields, even more preferably more than 50 fields.
S67. System according to any of the preceding system embodiments wherein the metadata comprises less than 100 fields, preferably less than 90 fields, even more preferably less than 70 fields.
S68. System according to any of the preceding system embodiments wherein the analyzing comprises a multidimensional analysis (MDA).
S69. System according to any of the preceding system embodiments wherein the first profile and/or the second profile is/are (a) vectorized profile(s) and the search in the general architecture is based on that vectorized profile(s).
S70. System according to any of the preceding system embodiments wherein the plurality of the datasets is sorted according to their relevance and/or weight and/or neighborhood and/or their similarity to the originating data or most recent originating data.
S71. System according to any of the preceding system embodiments which is configured for further displaying a representative summary of the plurality of the datasets sorted according to their relevance and/or neighborhood and/or their similarity to the originating data or most recent originating data.
S72. System according to any of the preceding system embodiments which is configured for further de-selecting and/or weighing of the datasets upon one or more clicks by the inputting component in pre-defined fields.
S73. System according to any of the preceding system embodiments which is configured for further displaying a representative summary of the plurality of the datasets sorted according to their relevance and/or neighborhood and/or their similarity to the originating data or most recent originating data and providing the option to out select datasets and/or to provide a quantitative value to their relevance.
S74. System according to the preceding system embodiment wherein the quantitative value is from 0 to 3 or from 0 to 4.
S75. System according to any of the two preceding system embodiments with a further value representing a de-selecting of a dataset.
S76. System according to any of the preceding system embodiments wherein a representative summary of the plurality of datasets comprises a plurality of metadata of the respective datasets.
S77. System according to any of the preceding system embodiments wherein the inputting component is configured to allow an expansion of any parts of the datasets upon a click by the inputting component.
S78. System according to any of the preceding system embodiments comprising an evaluating component for retrieving datasets corresponding to their quality and/or quantity and/or compatibility and/or similarity to a biological feature.
S79. System according to any of the preceding system embodiments with a sequencing component (5,12) for sequencing genetic samples and/or a sequencing analyzer (5,12) for analyzing sequences of genetic samples.
S80. System according to any of the preceding system embodiments comprising a high throughput sequencing component (5,12) for sequencing genetic samples with high throughput.
S81. System according to any of the preceding system embodiments comprising a metagenomics component (5,12) for providing and or sequencing metagenomics samples, such as environmental samples taken from soil, water and/or air.

Below, method embodiments will be discussed. These embodiments are abbreviated by the letter "M" followed by a number. Whenever reference is herein made to "method embodiments", these embodiments are meant.
M1. Method for an identification of biological feature in a biological resource, with the following steps:
   a. providing datasets in and/or from a resource database (1);
   b. analyzing similarity and/or compatibility of the datasets with biologically active features known;
   c. identifying the datasets that have the same, a compatible and/or have a minimum similarity to the biologically active features.
M2. Method for an identification of a biological feature in a biological resource, with the following steps:
   a. providing a plurality of datasets in and/or from a resource database (1);
   b. providing at least one first profile relevant for retrieving compatible and/or similar datasets to at least one preset feature;
   c. automatically approaching the resource database (1) and retrieving datasets from it according to the first profile;
   d. training the first profile on the basis of the datasets retrieved.
M3. Method for an identification of a biological feature in a biological resource, with the following steps:
   a. providing a plurality of datasets in and/or from a resource database (1);
   b. providing at least one first profile relevant for retrieving compatible datasets to at least one preset feature;
   c. wherein the first profile has been trained over time;
   d. automatically approaching the resource database (1) and retrieving datasets from it according to the first profile.
M4. Method comprising any combination of the preceding method embodiments.
M5. Method according to the preceding method embodiment with the further step of testing and/or analyzing the datasets retrieved for the same and/or a compatible and/or a similar biological activity of the features known.
M6. Method according to the preceding method embodiment with the further step of testing and/or analyzing the datasets retrieved for a similar biological activity of the features known.
M7. Method according to any of the preceding method embodiments wherein the similarity of the datasets to the biologically active features is preset by a minimum threshold value of similarity.
M8. Method according to any of the preceding method embodiments wherein the similarity of the datasets to the biologically active features is determined by a sliding minimum threshold value of similarity.
M9. Method according to any of the preceding method embodiments wherein the similarity of the datasets to the biologically active features is determined by a sliding minimum threshold value of similarity that is set by a machine learning algorithm.
M10. Method according to any of the preceding method embodiments wherein the similarity of the datasets to the biologically active features is determined by a sliding minimum threshold value of similarity that is set by a machine learning algorithm trained by results of testing and/or analyzing the datasets identified for the biological activity of the features known.
M11. Method according to any of the preceding method embodiments for an identification of a pharmaceutically and/or chemically active and/or industrially applicable biological feature in a biological resource, with the further step of automatically approaching the resource database (1) and retrieving datasets from it.
M12. Method according to any of the preceding method embodiments with the further step of automatically approaching the resource database (1) and retrieving datasets from it according to a first profile and selecting a plurality of first datasets being at least similar to or comprising this first profile.
M13. Method according to any of the preceding method embodiments with the further step of selecting one or more preferred datasets among the first datasets and automatically analyzing the preferred datasets according to at least a second profile.
M14. Method according to any of the preceding method embodiments with the further step of automatically approaching the resource database (1) and retrieving datasets from it according to a first profile and selecting a plurality of first datasets being at least similar to or comprising this first profile and selecting one or more of the first datasets and analyzing the preferred datasets according to at least a second profile.
M15. Method according to any of the preceding method embodiments with the further step of automatically approaching the resource database (1) and retrieving datasets from it according to a first profile and selecting a plurality of first datasets being at least similar to or comprising this first profile wherein the first profile comprises a subset of a sequence of the active feature known.
M16. Method according to any of the preceding method embodiments with the further step of automatically approaching the resource database (1) and retrieving datasets from it according to a first profile and selecting a plurality of first datasets being at least similar to or comprising this first profile and selecting one or more of the first datasets and analyzing the preferred datasets according to at least a second profile wherein the first profile is less complex than the second profile.
M17. Method according to the preceding method embodiment wherein pharmaceutically and/or chemically active and/or industrially applicable features are provided by an application database (2).
M18. Method according to the preceding method embodiment wherein the application database (2) comprises data of pharmaceutically and/or chemically active and/or industrially applicable features known.
M19. Method according to the preceding method embodiment wherein the application database (2) comprises data of pharmaceutically and/or chemically active and/or industrially applicable features and their applications known.
M20. Method according to any of the preceding method embodiments wherein the computing of the datasets from the resource database (1) is performed by automatically gathering dataset by dataset and/or sets of datasets from the resource database (1).
M21. Method according to any of the preceding method embodiments wherein the datasets from the resource database (1) are sequentially analyzed with the pharmaceutically and/or chemically active and/or industrially applicable features.
M22. Method according to any of the preceding method embodiments wherein the datasets from the resource database (1) are analyzed with the pharmaceutically and/or chemically active and/or industrially applicable features in parallel, at least in part.
M23. Method according to any of the preceding method embodiments wherein the analyzing is prioritized according to pre-set features.
M24. Method according to any of the preceding method embodiments wherein between the analyzing and providing for further use the similarity has at least a preset threshold value.
M25. Method according to any of the preceding method embodiments wherein threshold is a dynamic threshold that can vary according to a training of the analyzing step.
M26. Method according to any of the preceding method embodiments wherein the datasets provided are used for the formulation of pharmaceutically and/or chemically active and/or industrially applicable compounds.
M27. Method according to any of the preceding method embodiments with the further step of automatically analyzing the datasets on the bases of supervised machine learning.
M28. Method according to any of the preceding method embodiments with the further step of automatically analyzing the datasets on the bases of unsupervised machine learning.
M29. Method according to any of the preceding method embodiments wherein any training and/or machine learning is based on an explainable AI concept (XAI).
M30. Method according to any of the preceding method embodiments with the further step of automatically analyzing the datasets on the bases of at least two approaches.
M31. Method according to any of the preceding method embodiment with the further step of repeatedly selecting datasets and weighing the profiles of the first and second approaches and the classifiers from the supervised machine learning on the basis of the profiles and classifiers and providing an actual retrieving profile combining the weighted profiles and classifiers.
M32. Method according to the preceding method embodiment with the further step of automatically analyzing the preferred datasets according to the profiles of the first and second approaches and the classifiers of the supervised machine learning.
M33. Method according to any of the preceding method embodiments with the further step of repeatedly selecting datasets weighing the profiles of the first and second approaches and the classifiers on the basis of the profiles and classifiers and providing an actual retrieving profile combining the weighted profiles and classifiers.
M34. Method according to any of the preceding method embodiments wherein in the first approach is a first model and the second approach is a second model and the second model is different to the first model.
M35. Method according to any of the preceding method embodiments wherein the first approach profile comprises a numerical representation of the analysis of the originating data and/or the selected dataset(s) according to the first model.
M36. Method according to the preceding method embodiment wherein the second approach profile comprises a numerical representation of the analysis of the originating data (2) and/or the selected dataset(s) (10-12) according to the second model.
M37. Method according to any of the preceding method embodiments wherein the first approach comprises a content analytics model and the first approach profile comprises a first approach multidimensional representation.
M38. Method according to any of the preceding method embodiments wherein the second approach comprises a past distribution statistics model and the second approach profile comprises a second approach multidimensional representation.
M39. Method according to the two preceding method embodiments wherein the first and second approach multidimensional representations are vectorized.
M40. Method according to any of the preceding method embodiments with the further step of retrieving datasets from the database (4) on the basis on either one, two or all of the three models.
M41. Method according to the preceding method embodiment with the further step of weighing or gradually combining the three models on the basis of the last result of the selection component (5).
M42. Method according to any of the preceding method embodiments wherein the analyzing component can be activated by a soft key for refreshing the analysis.
M43. Method according to any of the preceding method with the further step of activating the analyzing by a soft key for refreshing the analysis after the selecting component (5) has been activated.
M44. Method according to the preceding method embodiment wherein the datasets each comprise at least one of datasets, callable units, metadata and content data and any combination thereof.
M45. Method according to any of the preceding method embodiments wherein the inputting of data is performed automatically or semi-automatically.
M46. Method according to any of the preceding method embodiments wherein the inputting of data is supported by a graphical user interface (GUI).
M47. Method according to any of the preceding method embodiments wherein the inputting of data is performed by initiating the pulling of data from a data base by entering a code.
M48. Method according to any of the preceding method embodiments wherein the database comprises more than a million datasets, preferably more than 10 million datasets and most preferably more than 100 million datasets.
M49. Method according to any of the preceding method embodiments wherein the machine learning establishes classifiers on the basis of the profiles by forming random decision trees on the basis of the features of the profiles and determining relevant decision trees from less relevant decision trees on the basis of profiles relevant for retrieving compatible datasets to originating data (2).
M50. Method according to any of the preceding method embodiments wherein any retrieving of compatible datasets makes the analyzing component vto analyze the originating data or any subsequent originating data on the basis of the first approach, the second approach and the classifiers.
M51. Method according to any of the preceding method embodiments wherein the datasets each comprise at least one of datasets, callable units, metadata and content data and any combination thereof.
M52. Method according to any of the preceding method embodiments steps of inputting originating data or grading datasets automatically or semi-automatically.
M53. Method according to the preceding method embodiment wherein an inputting component (1) can initiate the method upon entry of an individual password.
M54. Method according to any of the preceding method embodiments with the step of repeating the initiating of the retrieving of compatible datasets any time on the most recent corresponding profile(s) in the analytics database.
M55. Method according to any of the preceding method embodiments further comprising controlling a plurality of inputting components so that they are configured or can be configured upon entry of a code to share data in the analytics database assigned to the inputting components.
M56. Method according to any of the preceding method embodiments with the step of repeating retrieving compatible datasets based on the profiles in the analytics database.
M57. Method according to the two preceding method embodiments with the step of repeating retrieving compatible data by another inputting component than the previous one.
M58. Method according to any of the preceding method embodiments wherein the originating data comprises metadata and content data.
M59. Method according to the preceding method embodiment wherein the originating data comprises a code that is part of the metadata and wherein the database comprises the other metadata and/or content data that corresponds to the code of the originating data.
M60. Method according to the preceding method embodiment with the step of pulling the other metadata and/or the content data from the database on the basis of the code.
M61. Method according to any of the preceding method embodiments wherein the datasets each comprise metadata and content data.
M62. Method according to the preceding method embodiment wherein the features of the profiles correspond to portions of the metadata of the datasets.
M63. Method according to any of the preceding method embodiments wherein the originating data is provided by the inputting component in encoded fashion in a code and the analyzing component is adapted to gather the complete originating data from the database based on that code.
M64. Method according to any of the preceding method embodiments wherein the code is an ID number, such as a document number, and the complete originating data comprises a document.
M65. Method according to the preceding method embodiment wherein the complete originating data (2) comprises meta data and content data.
M66. Method according to any of the preceding method embodiments wherein the metadata comprises more than 5 fields, preferably more than 10 fields, even more preferably more than 50 fields.
M67. Method according to any of the preceding method embodiments wherein the metadata comprises less than 100 fields, preferably less than 90 fields, even more preferably less than 70 fields.
M68. Method according to any of the preceding method embodiments wherein the analyzing comprises a multidimensional analysis (MDA).
M69. Method according to any of the preceding method embodiments wherein the first profile and/or the second profile is/are (a) vectorized profile(s) and the search in the general architecture is based on that vectorized profile(s).
M70. Method according to any of the preceding method embodiments wherein the plurality of the datasets is sorted according to their relevance and/or weight and/or neighborhood and/or their similarity to the originating data or most recent originating data.
M71. Method according to any of the preceding method embodiments with the step of displaying a representative summary of the plurality of the datasets sorted according to their relevance and/or neighborhood and/or their similarity to the originating data (2) or most recent originating data (2).
M72. Method according to any of the preceding method embodiments with the further step of de-selecting and/or weighing of the datasets upon one or more clicks by the inputting component in pre-defined fields.
M73. Method according to any of the preceding method embodiments with the further step of displaying a representative summary of the plurality of the datasets (5-7) sorted according to their relevance and/or neighborhood and/or their similarity to the originating data (2) or most recent originating data (2) and providing the option to out select datasets and/or to provide a quantitative value to their relevance.
M74. Method according to the preceding method embodiment wherein the quantitative value is from 0 to 3 or from 0 to 4.
M75. Method according to any of the two preceding method embodiments with a further value representing a de-selecting of a dataset.
M76. Method according to any of the preceding method embodiments wherein a representative summary of the plurality of datasets comprises a plurality of metadata of the respective datasets.
M77. Method according to any of the preceding method embodiments wherein the inputting component is configured to allow an expansion of any parts of the datasets upon a click by the inputting component.
M78. Method according to any of the preceding method embodiments with the step of evaluating the retrieving of datasets corresponding to their quality and/or quantity and/or compatibility and/or similarity to a biological feature.
M79. Method according to any of the preceding method embodiments with the step of retrieving datasets corresponding to their quality and/or quantity and/or compatibility and/or similarity to a biological feature.
M80. Method according to any of the preceding method embodiments with the step of sequencing genetic samples and/or a analyzing sequences of genetic samples.
M81. Method according to any of the preceding method embodiments with the step of sequencing genetic high throughput sequencing component (5,12) for sequencing genetic samples with high throughput.
M82. Method according to any of the preceding method embodiments with the step of providing and or sequencing metagenomics samples, such as environmental samples taken from soil, water and/or air.

Below, use embodiments will be discussed. These embodiments are abbreviated by the letter "U" followed by a number. Whenever reference is herein made to "use embodiments", these embodiments are meant.
U1. Use of the system according to any of the preceding system embodiment for an identification of a biological feature in a biological resource by applying the system according to any of the preceding system embodiments.
U2. Use of the method according to any of the preceding method embodiments for an identification of a biological feature in a biological resource by carrying out the method according to any of the preceding method embodiments.
U3. Use of the method according to any of the preceding method embodiments for an identification of a biological feature in a biological resource in the field of red biotechnology by carrying out the method according to any of the preceding method embodiments.
U4. Use of the method according to any of the preceding method embodiments for an identification of a biological feature in a biological resource in the field of green biotechnology by carrying out the method according to any of the preceding method embodiments.
U5. Use of the method according to any of the preceding method embodiments for an identification of a biological feature in a biological resource in the field of white biotechnology by carrying out the method according to any of the preceding method embodiments.
U6. Use of the method according to any of the preceding method embodiments for an identification of a biological feature in a biological resource in the field of yellow biotechnology by carrying out the method according to any of the preceding method embodiments.
U7. Use of the method according to any of the preceding method embodiments for an identification of a biological feature in a biological resource in the field of blue biotechnology by carrying out the method according to any of the preceding method embodiments.
U8. Use of the method according to any of the preceding method embodiments for an identification of a biological feature in a biological resource in the field of grey biotechnology by carrying out the method according to any of the preceding method embodiments.
U9. Use of the method according to any of the preceding method embodiments for an identification of a biological feature in a biological resource in the field of gold biotechnology by carrying out the method according to any of the preceding method embodiments.

Below, computer related product embodiments will be discussed. These embodiments are abbreviated by the letter "C" followed by a number. Whenever reference is herein made to "computer related product embodiments", these embodiments are meant.
C1. A computer related product with a program that is configured for carrying out the method according to any of the preceding method embodiments.

Below, diagnostic embodiments will be discussed. These embodiments are abbreviated by the letter "D" followed by a number. Whenever reference is herein made to "diagnostic embodiments", these embodiments are meant.
D1. Method of a diagnostic treatment of a patient with a pharmaceutical component that has been provided by a method according to any of the preceding method embodiments.
D2. Method of a diagnostic treatment of a patient with a pharmaceutical component that has been provided by a system according to any of the preceding system embodiments.

Below, therapeutic embodiments will be discussed. These embodiments are abbreviated by the letter "T" followed by a number. Whenever reference is herein made to "therapeutic embodiments", these embodiments are meant.
T1. Method of a therapeutic treatment of a patient with a pharmaceutical component that has been provided by a method according to any of the preceding method embodiments.
T2. Method of a therapeutic treatment of a patient with a pharmaceutical component that has been provided by a system according to any of the preceding system embodiments.

The present invention will now be described with reference to the accompanying drawings, which illustrate embodiments of the invention. These embodiments should only exemplify, but not limit, the present invention.

### Figure Description

- Fig. 1: schematically exemplifies a scheme in accordance with an embodiment according to the invention.
- Fig. 2: shows another embodiment in accordance with the present invention.

### Description of preferred embodiments as exemplified in the figures

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

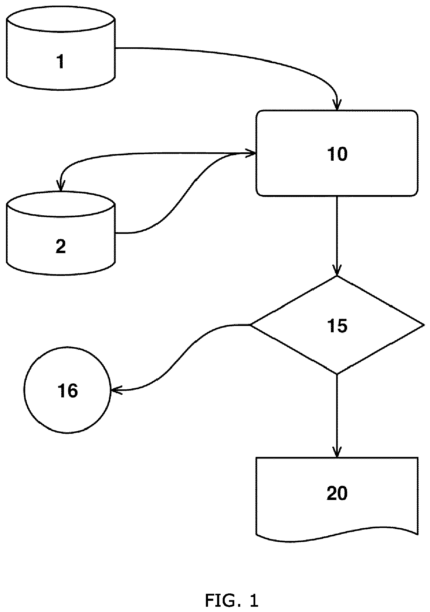

Fig. 1 provides a schematic view onto a computing component 10 in connection with a biological resource database 1 and an application database 2. The application database comprises a collection of pharmaceutically and/or chemically active and/or industrially applicable compounds and their field(s) of application already known.

As can be seen, the computing component is able to read data or datasets from the biological resource database 1 and to access the application database 2. Whenever a bio dataset, such as a DNA sequence, is fed into the computing component 10, the application database is consulted for similar or equal datasets. This can be done by a similarity comparison, such as vectorization. Anyhow, there is a comparator 15 affiliated to the computing component 10. The comparator 15 can be arranged in a separate or even remote manner to the computing component but can also be integrated into the computing component 10.

In the comparator the similarity of a or each dataset from the resource database 1 can be analyzed with a pharmaceutically and/or chemically active and/or industrially applicable compound in the application database 2. As mentioned before the comparator 15 can be provided with a trained comparator or can be used in order to train the comparator 15.

In case the comparator 15 decides that the pharmaceutical activity of a dataset from the resource database 1 is below a pre-set similarity threshold analyzed to a or each pharmaceutical compound in the application database 2 it is not considered any further. This is symbolized with the end of consideration 16. In case it is on or above this threshold, pharmaceutical activity is assumed and the respective resource dataset analyzed is provided to an interface 20 as a candidate for pharmaceutical activity. The respective datasets considered potentially relevant can then further provided into an analysis stage (not shown).

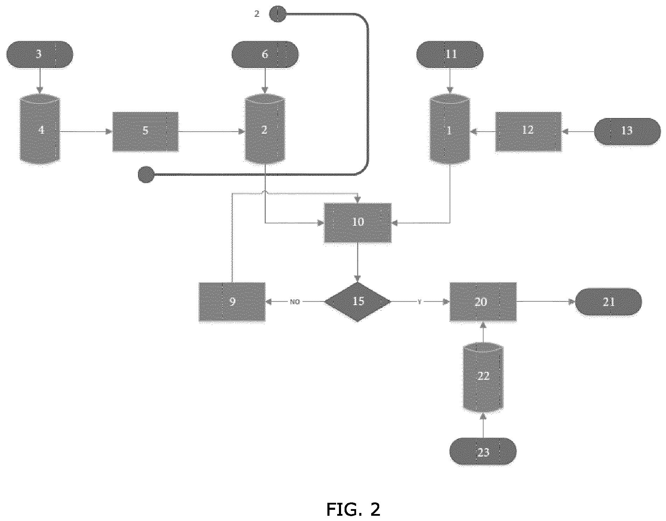

Fig. 2 shows more components or elements in accordance with the present invention. This does by no means mean that all those elements are necessary for the present invention. Also, a subset of the components or elements is within comprised by the present invention.

A feature database 4 can be provided that has all or just a part of the information for the features. E.g., they can comprise the CPNO database, the NPACT database, or any database containing market information. This can be trained. In a first sequencer 5 or a sequence analyzer 5 can provide the clusters or families of genes, biosynthetic information, etc. into feature or application database 2. It can be also fed with Big-FA, UniProtKB etc. These elements belong to the training of functional gene sequences. They can be also contained in the application database or server 2 as described before.

The resource database 1 can further deliver its information or data. This resource database 1 can be fed with data mined in a miner 11 and/or sample sequence data originating from new or known samples 13 of biological origin, such as scouting samples. These samples can be sequenced in a second sequencer 12 and delivered to the resource database 1. Examples are EggNOG, InterPro2GO, KEGG, SEED, dbGaP, OMIM and/or continuously and/or sequentially generated or delivered.

The data from the databases 1, 2 can be used and analyzed in the AI or ML processing component or computing component 10. The result can then go to comparator 15 where a comparison can be done. In case of a presence of a function or feature function (depicted with Y) the information goes further to interface 20. This interface 20 can be an active or a passive component. The interface can be provided with market data from a market information database 22 that comprises the data from a market data training component 23.

In an analyzer 21 the result can be analyzed and/or tested.

In case no function or feature function can be determined this is fed back by an AI or ML validation component 9 in order to train the computing component accordingly.

Reference numbers and letters appearing between parentheses in the claims, identifying features described in the embodiments and illustrated in the accompanying drawings, are provided as an aid to the reader as an exemplification of the matter claimed. The inclusion of such reference numbers and letters is not to be interpreted as placing any limitations on the scope of the claims.

The term "at least one of a first option and a second option" is intended to mean the first option or the second option or the first option and the second option.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. System for an identification of biological feature in a biological resource comprising:
a. a resource database (1) configured for providing datasets;
b. an analyzing component (10,15)
i. configured for analyzing similarity and/or compatibility of the datasets with biologically active features known; and
ii. for identifying the datasets that have the same, a compatible and/or have a minimum similarity to the biologically active features.

2. System according to the preceding claim wherein the analyzing component (10,15) comprises a computing component (10) that is configured for providing at least one first profile relevant for retrieving compatible datasets to at least one preset feature and for automatically approaching the resource database (1) and retrieving datasets from it according to the first profile and for training the first profile on the basis of the datasets retrieved.

3. System according to the preceding claim wherein the first profile is trained over time.

4. System according to any of the preceding claims with an analyzing component (10,15) configured for testing and/or analyzing the datasets retrieved for the same and/or a compatible and/or a similar biological activity of the features known.

5. System according to any of the preceding claims wherein the similarity of the datasets to the biologically active features is preset by a minimum threshold value of similarity.

6. System according to any of the preceding claims wherein the similarity of the datasets to the biologically active features is determined by a sliding minimum threshold value of similarity that is set by a machine learning algorithm.

7. System according to any of the preceding claims for an identification of a pharmaceutically and/or chemically active and/or industrially applicable biological feature in a biological resource, with the computing component (10) configured for automatically approaching the resource database (1) and retrieving datasets from it.

8. System according to any of the preceding claims with an analyzing component (10,15) configured for automatically approaching the resource database (1) and retrieving datasets from it according to a first profile and selecting a plurality of first datasets being at least similar to or comprising this first profile.

9. System according to any of the preceding claims with a comparator (10,15) configured for selecting one or more preferred datasets among the first datasets and automatically analyzing the preferred datasets according to at least a second profile.

10. System according to the preceding claim wherein the first profile is less complex than the second profile.

11. Method for an identification of biological feature in a biological resource, with the following steps:
a. providing datasets in and/or from a resource database (1);
b. analyzing similarity and/or compatibility of the datasets with biologically active features known;
c. identifying the datasets that have the same, a compatible and/or have a minimum similarity to the biologically active features.

12. Method according to the preceding claim with the further steps of providing at least one first profile relevant for retrieving compatible and/or similar datasets to at least one preset feature, automatically approaching the resource database (1) and retrieving datasets from it according to the first profile and training the first profile on the basis of the datasets retrieved.

13. Method according to the preceding claim wherein the first profile has been trained over time.

14. Method according to the preceding claim with the further step of testing and/or analyzing the datasets retrieved for the same and/or a compatible and/or a similar biological activity of the features known.

15. Method according to the preceding claim with the further step of testing and/or analyzing the datasets retrieved for a similar biological activity of the features known.

16. Method according to any of the preceding claims wherein the similarity of the datasets to the biologically active features is determined by a sliding minimum threshold value of similarity.

17. A computer related product with a program that is configured for carrying out the method according to any of the preceding method embodiments.
